# FASCICULE DE BREVET EUROPEEN

(11) **EP 3 899 056 B1**
(45) Date de publication et mention de la délivrance du brevet: **18.01.2023**
(21) Numéro de dépôt: 19845713.7
(22) Date de dépôt: 18.12.2019
(51) Int. Cl.: C14C 1/00, A61K 8/36, A61Q 5/02, A61K 8/365, A61K 8/46, A61K 8/67, A61Q 5/04, A61Q 9/04

(54) **COMPOSITION D'ACIDE THIOGLYCOLIQUE À FAIBLE ODEUR**
GERUCHSARME THIOGLYKOLSÄUREZUSAMMENSETZUNG
LOW-ODOUR THIOGLYCOLIC ACID COMPOSITION

(30) Priorité: 21.12.2018 FR 1873828
(43) Date de publication de la demande: 27.10.2021
(73) Titulaire: ARKEMA FRANCE, 92700 Colombes (FR)
(72) Inventeur: MONGUILLON, Bernard, 92700 COLOMBES (FR); SKOWRON, Pierre-Thomas, 64170 LACQ (FR)
(74) Mandataire: Arkema Patent
(86) Numéro de dépôt international: PCT/FR2019/053145
(87) Numéro de publication internationale: WO 2020/128321

(56) Documents cités:
- GB-A- 2 114 988
- CHRISTINA L. BURNETT ET AL: "Final Amended Report on the Safety Assessment of Ammonium Thioglycolate, Butyl Thioglycolate, Calcium Thioglycolate, Ethanolamine Thioglycolate, Ethyl Thioglycolate, Glyceryl Thioglycolate, Isooctyl Thioglycolate, Isopropyl Thioglycolate, Magnesium Thioglycolate, Methyl Thioglycolate, Potassium Thio", INTERNATIONAL JOURNAL OF TOXICOLOGY, vol. 28, no. 4_suppl, 1 juillet 2009 (2009-07-01), pages 68-133, XP055618538, US ISSN: 1091-5818, DOI: 10.1177/1091581809339890

## Description

La présente invention concerne une composition d'acide thioglycolique (ci-après appelé « ATG ») à faible odeur, son procédé de préparation, ainsi que les utilisations de ladite composition.

L'acide thioglycolique, de formule suivante :

HS-CH₂-COOH

ainsi que ses sels et esters, est utilisé dans de nombreux domaines tels que la cosmétique (par exemple dans les shampoings, les compositions pourfrisage ou défrisage des cheveux et les crèmes dépilatoires), le traitement du cuir, les solutions de nettoyage ou encore dans la préparation de polymères en tant qu'agent de transfert de chaîne. L'utilisation d'acide thioglycolique présente toutefois un inconvénient dû à son odeur désagréable, caractérisée notamment par une odeur soufrée et acide, voire rance, associée à une odeur de grillé et/ou de brûlé. De plus, dans certaines conditions, l'acide thioglycolique peut se décomposer en hydrogène sulfuré (H₂S) et autres composés tels que des mercaptans légers très malodorants.

Cette odeur est gênante lors de la manipulation de l'ATG et peut se retrouver jusque dans les produits finis en contenant, ce qui représente un inconvénient majeur à son utilisation, en particulier dans le domaine cosmétique.

Pour contrer cette odeur désagréable, plusieurs stratégies ont été employées par les industriels : masquer l'odeur de l'ATG à l'aide d'agents masquant d'odeur ou diminuer le relargage d'H₂S et/ou de mercaptans légers pouvant être responsables de l'odeur.

On peut ainsi citer par exemple, l'utilisation :
- d'agents masquant d'odeur de type substances naturelles, extraits de substances naturelles ou bases parfumantes ;
- d'adsorbants tels que le charbon ou les zéolithes ou de cyclodextrines permettant de diminuer la quantité d'H₂S et/ou de mercaptans légers relargués ; ou
- d'additifs spécifiques permettant également de diminuer la quantité d'H₂S et/ou de mercaptans légers relargués tels que les polyphénols ou les oxydes métalliques.

Ainsi, la plupart des solutions proposées s'intéressent uniquement au masquage ou à l'adsorption des composés malodorants. Ces solutions ne prennent pas en compte le fait que l'acide thioglycolique obtenu industriellement contient des impuretés, dont certaines peuvent être en partie responsables de l'odeur désagréable de l'ATG.

GB 2 114 988 A décrit une composition contenant un acide carboxylique organique, tel que l'acide thiolactique et de l'acide thioglycolique avec un ratio molaire compris entre 0,0004 et 0,1.

Il existe donc un besoin pour une composition d'acide thioglycolique à faible odeur. Plus particulièrement, il existe un besoin pour une composition d'acide thioglycolique à faible odeur pouvant être obtenue de façon industrielle. Il existe également un besoin pour une composition d'acide thioglycolique dont l'odeur désagréable est masquée ou diminuée, voire supprimée, en particulier dans les produits finis en contenant.

La présente invention a donc pour but de proposer des compositions comprenant de l'acide thioglycolique à faible odeur. Plus particulièrement, la présente invention a pour but de proposer des compositions à faible odeur comprenant un acide thioglycolique obtenu par un procédé de préparation industriel, plus particulièrement par un procédé de préparation industriel utilisant l'acide monochloroacétique (AMCA) ou ses sels.

La présente invention a également pour but de proposer une composition d'acide thioglycolique à faible odeur, notamment utile dans le domaine cosmétique, le traitement du cuir, les solutions de nettoyage, ou encore dans la préparation de polymères en tant qu'agent de transfert de chaîne.

Ainsi, la présente invention concerne une composition **C** comprenant :
- au moins un acide organique **A,** linéaire ou cyclique, comprenant de 1 à 3 fonction(s) acide carboxylique et comprenant une chaîne hydrocarbonée saturée ou insaturée ; linéaire ou ramifiée ou cyclique contenant de 1 à 10 atome(s) de carbone ;
   lesdits atome(s) de carbone étant éventuellement substitué(s) par un ou plusieurs substituant(s) choisi(s) parmi -OH et =O ; ou l'un de ses sels ;
- une composition **B** comprenant :
   a) de l'acide thioglycolique, ou l'un de ses sels ou de ses esters ; et
   b) du thioglycolate d'isopropyle (IPTG) en une quantité comprise entre strictement supérieur à 0 et 130 ppm, de préférence entre 5 et 80 ppm, et plus préférentiellement entre 30 et 70 ppm ; par exemple entre 20 et 70 ppm ; et
- éventuellement un solvant **S** ;
lesdits acide(s) organique(s) A et acide thioglycolique étant en des proportions telles que le ratio molaire (acide(s) organique(s) A/acide thioglycolique) est compris entre 0,0004 et 0,1 ; de préférence entre 0,0004 et 0,004.

Contrairement aux solutions connues de l'art antérieur telles que mentionnées ci-dessus, les présents inventeurs ont découvert que la combinaison d'au moins un acide organique ayant 1, 2 ou 3 fonction(s) -C(O)OH, avec de l'ATG obtenu par un procédé industriel permettait de masquer, diminuer voire supprimer l'odeur de ce dernier. Plus particulièrement, la composition C selon l'invention a une odeur moins intense et un caractère hédonique plus important qu'une composition B d'acide thioglycolique telle que définie ci-dessus. Ainsi, l'odeur soufrée et/ou de grillé et/ou de brûlé de la composition B d'ATG est notamment masquée, diminuée voire supprimée grâce à l'ajout d'au moins un acide organique A tel que défini ci-dessus. En particulier, l'odeur soufrée de la composition B d'ATG est masquée, diminuée voire supprimée grâce à l'ajout d'au moins un acide organique A tel que défini ci-dessus.

Selon un mode réalisation, une telle composition B correspond à une composition d'acide thioglycolique obtenue industriellement, par exemple en utilisant l'AMCA ou l'un de ses sels selon le procédé décrit ci-dessous.

Ainsi, la préparation industrielle de l'acide thioglycolique est décrite dans la publication « Thioglycolic acid », Y. Labat, Kirk-Othmer Encyclopedia of Chemical Technology, Fourth Edition, vol. 24, 1997, ISBN 0-471-52693-2, en particulier en pages 3-6 « Manufacturing, Processing, and Storage »). L'acide thioglycolique est produit par la réaction de l'acide monochloroacétique (AMCA) ou l'un de ses sels avec des sulfhydrates alcalins comme NaSH ou NH₄SH en milieu aqueux. Le mélange réactionnel est ensuite acidifié de manière à libérer l'acide thioglycolique. Ces deux étapes réactionnelles sont résumées dans les équations suivantes :

Cl-CH₂-COOH + 2 XSH -> HS-CH₂-COO⁻X⁺ + XCl + H₂S avec X = NH₄ ou Na (1)

HS-CH₂-COO⁻X⁺ + HCl -> HS-CH₂-COOH + XCl (2)

L'acide thioglycolique obtenu est ensuite extrait de la solution aqueuse avec un solvant organique, préférentiellement de type éther comme l'éther diisopropylique. Le solvant organique est évaporé puis l'acide thioglycolique est purifié par distillation sous vide. L'acide thioglycolique peut subir ensuite un étêtage pour éliminer les composés légers. Enfin, l'acide thioglycolique peut subir un équeutage afin d'éliminer les composés lourds. L'éther diisopropylique (DIPE ou IPE) est un solvant d'extraction communément utilisé en industrie. Dans le procédé de production d'ATG, l'IPE est en contact avec de l'acide chlorhydrique et peut subir un clivage acide pour former de l'isopropanol. Par estérification de l'isopropanol avec l'ATG, on forme le thioglycolate d'isopropyle (IPTG).

A l'issue de ce procédé industriel de fabrication, la composition à base d'acide thioglycolique obtenue peut donc comprendre de l'IPE et de l'IPTG.

### Définitions

On entend notamment par « composition d'acide thioglycolique à faible odeur » une composition C telle que selon l'invention. En particulier, ladite composition C a une odeur présentant une intensité moindre ou égale et un caractère hédonique supérieur ou égal à une composition d'acide thioglycolique à forte odeur telle que définie ci-après.

On entend notamment par « composition d'acide thioglycolique à forte odeur » une composition B telle que selon l'invention. En particulier, ladite composition B d'acide thioglycolique est obtenue suite à un procédé industriel utilisant l'AMCA comme produit de départ.

En particulier, la composition C selon l'invention présente une odeur soufrée (ou note soufrée) masquée, diminuée ou supprimée par rapport à une composition B d'acide thioglycolique ne comprenant pas d'acide organique A tel que défini ci-dessus.

Par « odeur », on entend la perception olfactive d'un produit, ici des compositions B et C, qui peut être caractérisée par analyse sensorielle. L'intensité, le caractère hédonique et la description de l'odeur sont des paramètres habituellement utilisés dans la caractérisation sensorielle d'une odeur. La détermination de la concentration d'une odeur par olfactométrie dynamique peut se faire selon la norme EN 13725 (version Octobre 2003).

Par « intensité olfactive », on entend par exemple la mesure d'intensité basée sur la norme allemande VDI 3882 part 1. Le panel de personnes sélectionnées peut se faire selon la norme EN 13725. L'intensité peut être évaluée sur une échelle de 0 à 5 (5 très fort, 4 fort, 3 moyen, 2 faible, 1 très faible, 0 pas détectable). Selon un mode de réalisation, les compositions selon l'invention présentent une intensité moyenne entre 2 et 4, d'environ 3, par exemple entre 2,5 et 3,5.

Par « caractère hédonique », on entend le caractère agréable ou désagréable d'une odeur. Par exemple, la mesure du caractère hédonique peut être basée sur la norme allemande VDI 3882 part 2. Le panel de personnes sélectionnées peut se faire selon la norme EN 13725. Le caractère hédonique peut être évalué sur une échelle de -4 à +4 (+4 extrêmement agréable, +3 très agréable, +2 agréable, +1 faiblement agréable, 0 neutre, -1 faiblement désagréable, -2 désagréable, -3 très désagréable et -4 extrêmement désagréable). Selon un mode de réalisation, le caractère hédonique des compositions selon l'invention est compris entre -1 et +1, par exemple entre -0,3 et +1,5.

Par «fonction acide carboxylique », on entend un groupement -C(O)OH. Par acide organique A cyclique, on entend un acide organique dont la fonction acide carboxylique est cyclisée, par exemple l'acide ascorbique.

Le terme « (Ci-Cio)alkyle » désigne des hydrocarbures aliphatiques saturés, qui peuvent être linéaires ou ramifiés et comprennent de 1 à 10 atome(s) de carbone. De préférence, lesdits alkyles comprennent de 1 à 5 atome(s) de carbone, voire de 1 à 4 atome(s) de carbone. Par « ramifié », on entend qu'un groupement alkyle est substitué sur la chaîne alkyle principale. Les alkyles préférés selon l'invention sont le méthyle, l'éthyle, le propyle et le butyle.

Le terme « (C₁-C₁₀)alcényle » se réfère à un (Ci-Cio)alkyle comprenant au moins une double liaison, de préférence une seule double liaison.

Le terme "(C₃-C₁₀)cycloalkyle" désigne des hydrocarbures aliphatiques saturés cycliques qui comprennent de 3 à 10 atomes de carbone, en particulier le cyclopropyle ou le cyclohexyle.

Le terme « (C₆-C₁₀)aryle » désigne des hydrocarbures aromatiques monocycliques, bicycliques ou tricycliques, en particulier le phényle et le naphtyle.

Les sels de l'acide organique A et les sels et esters de l'acide thioglycolique sont connus de l'homme du métier. Les esters de l'ATG sont par exemple les esters de méthyle, éthyle, glycérol ou sorbitol. Par exemple, pour l'acide thioglycolique, on peut envisager les sels de Na, Ca, K, NH₄ et de monoéthanolamine. Selon un mode de réalisation, les acides organiques A sont choisis parmi les composés de formule (I') suivante : 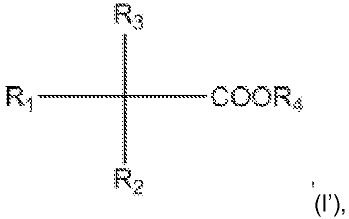 dans laquelle, R₁, R₂ et R₃ sont tels que définis pour la formule (I) et R₄ est choisi parmi le groupe constitué de H, Na, Ca, K et NH₄.

Selon un mode de réalisation préféré, ladite composition C ne comprend pas de sels d'acide organique A ni de sels d'acide thioglycolique.

Par ppm on entend partie par million par exprimer une fraction massique (1 ppm = 1 mg/kg).

### Compositions C selon l'invention

La présente invention a pour objet des compositions C d'acide thioglycolique telles que définies ci-dessus, et de préférence à faible odeur.

Selon un mode de réalisation, lesdits acide(s) organique(s) A et acide thioglycolique sont en des proportions telles que le ratio molaire (acide(s) organique(s) A/acide thioglycolique) est compris entre 0,0005 et 0,005 ; de préférence entre 0,0007 et 0,008.

Selon un mode de réalisation, le ratio molaire (acide(s) organique(s) A/acide thioglycolique) est compris entre 0,0008 et 0,008.

Selon un mode de réalisation, ladite composition C comprend entre 0,1% et 99,9%, de préférence entre 50% et 99,9% en poids d'acide thioglycolique par rapport au poids total de la composition C. Plus préférentiellement, la composition C selon l'invention comprend environ 70%, 80%, 85%, 97%, 98%, 99% ou 99,9%, de préférence 80% ou 99% en poids d'acide thioglycolique par rapport au poids total de ladite composition C. Par exemple, la composition C comprend au moins 99% en poids d'acide thioglycolique par rapport au poids total de ladite composition C.

### Acide organique A

Selon un mode de réalisation, la composition C comprend un acide organique A.

Selon un mode de réalisation, la composition C comprend au moins un acide organique A, linéaire ou cyclique, constitué de 1 à 3 fonction(s) acide carboxylique et d'une chaîne hydrocarbonée saturée ou insaturée ; linéaire ou ramifiée ou cyclique contenant de 1 à 10 atome(s) de carbone ;
lesdits atomes de carbone étant éventuellement substitué(s) par un ou plusieurs substituant(s) choisi(s) parmi -OH et =O, ou l'un de ses sels.

Selon un mode de réalisation, la composition C comprend au moins un acide organique A linéaire, comprenant 1 fonction acide carboxylique et comprenant une chaîne hydrocarbonée saturée ; linéaire, ramifiée ou cyclique contenant de 1 à 10 atome(s) de carbone ; lesdits atome(s) de carbone étant éventuellement substitué(s) par un ou plusieurs substituant(s) choisis parmi -OH et =O, ou l'un de ses sels.

Selon un mode de réalisation, ledit au moins un acide organique A est l'acide ascorbique ou un acide organique de formule générale (I) suivante : 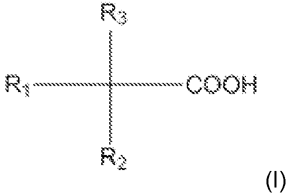 dans laquelle :
▪ R₁ est choisi parmi le groupe constitué de :
   - H, -OH, (Ci-Cio)alkyle et (C₂-C₁₀)alcényle ;
   lesdits (C₁-C₁₀)alkyle et (C₂-C₁₀)alcényle étant éventuellement substitué(s) par au moins un substituant choisi parmi le groupe constitué de : -OH, -C(O)OH et =O ;
▪ R₂ est -H, -OH ou (Ci-Cio)alkyle ;
▪ R₃ est H ;
   ou R₂ et R₃ forment ensemble avec l'atome de carbone auxquels ils se rattachent un groupement C=O ;
   ou R₁ et R₂ forment ensemble avec l'atome de carbone auxquels ils se rattachent un groupement (C₃-C₁₀)cycloalkyle ou (C₆-C₁₀)aryle,
   lesdits groupements (C₃-C₁₀)cycloalkyle et (C₆-C₁₀)aryle étant éventuellement substitué(s) par au moins un substituant choisi parmi le groupe constitué de :

      -OH, -C(O)OH et =O ;
ou l'un de ses sels.

Selon un mode de réalisation, R₁ est choisi dans le groupe constitué de : -H, -OH et (C₁-C₈)alkyle ; lesdits (C₁-C₈)alkyles étant éventuellement substitué(s) par un, deux, trois ou quatre substituant(s) choisi(s) parmi le groupe constitué de : -OH, -C(O)OH et =O.

Selon un mode de réalisation, R₂ est -H ou -OH. Selon un mode de réalisation, R₂ et R₃ sont H.

Selon un mode de réalisation préféré, R₁ est un (Ci-Cio)alkyle éventuellement substitué par au moins un substituant choisi dans le groupe constitué de : -OH, -C(O)OH et =O et R₃ est H.

Selon un mode de réalisation, ledit au moins un acide organique A est un acide organique de formule générale (I) telle que définie ci-dessus.

Selon un mode de réalisation, l'acide organique A est choisi dans le groupe constitué de l'acide acétique, l'acide glycolique, l'acide lactique, l'acide propionique, l'acide citrique, l'acide salicylique, l'acide cyclohexanecarboxylique, l'acide 2-éthylbutyrique, l'acide pyruvique, l'acide lévulinique, l'acide ascorbique, l'acide acétonedicarboxylique, l'acide 2-céto-L-gluconique, et leurs mélanges.

Selon un mode de réalisation, l'acide organique A est choisi dans le groupe constitué de l'acide acétique, l'acide glycolique, l'acide lactique, l'acide propionique, l'acide citrique, l'acide salicylique, l'acide cyclohexanecarboxylique, l'acide 2-éthylbutyrique, l'acide pyruvique, l'acide lévulinique, l'acide acétonedicarboxylique, l'acide 2-céto-L-gluconique, et leurs mélanges.

Selon un mode de réalisation, l'acide organique A est choisi dans le groupe constitué de l'acide acétique, l'acide glycolique, l'acide lactique, l'acide propionique, l'acide citrique, l'acide salicylique, l'acide cyclohexanecarboxylique, l'acide 2-éthylbutyrique, l'acide pyruvique, l'acide lévulinique, l'acide ascorbique, l'acide acétonedicarboxylique, et leurs mélanges.

Selon un mode de réalisation, l'acide organique A est choisi dans le groupe constitué de l'acide acétique, l'acide propionique, l'acide 2-éthylbutyrique, l'acide pyruvique, l'acide ascorbique, l'acide acétonedicarboxylique, l'acide céto-L-gluconique, l'acide citrique et leurs mélanges.

Selon un mode réalisation, l'acide organique A est choisi dans le groupe constitué de l'acide acétique, l'acide propionique, l'acide 2-éthylbutyrique, l'acide pyruvique, l'acide acétonedicarboxylique, et leurs mélanges.

Préférentiellement, l'acide organique A est choisi parmi l'acide acétique et l'acide 2-éthylbutyrique, de préférence l'acide acétique.

Selon un autre mode de réalisation, l'acide organique A n'est ni l'acide ascorbique, ni l'acide 2-céto-L-gluconique ou leurs sels. Selon un autre mode de réalisation, l'acide organique A n'est ni l'acide ascorbique, ni l'acide 2-céto-L-gluconique, ni l'acide érythorbique ou leurs sels.

Selon un mode de réalisation, la composition C ne comprend pas d'agents masquant d'odeur autre que l'acide organique A tel que défini ci-dessus. Par exemple, la composition C ne comprend pas de base parfumante, par exemple de substances naturelles ou extraits de substances naturelles.

### Composition B

La composition B est comprise dans la composition C selon l'invention. La composition C peut être considérée comme une composition B additivée afin de masquer, diminuer voire de supprimer son odeur.

Selon un mode de réalisation, la composition B comprend :
a) de l'acide thioglycolique ou l'un de ses sels ;
b) du thioglycolate d'isopropyle (IPTG) en une quantité comprise entre strictement supérieur à 0 et 130 ppm, de préférence entre 5 et 80 ppm, et plus préférentiellement entre 30 et 70 ppm ; et
c) de l'éther diisopropylique (IPE) en une quantité comprise entre 5 et 100 ppm, de préférence entre 10 et 80 ppm.

Selon un mode de réalisation, la composition B comprend :
a) de 0,1% à 99,9%, de préférence de 50% à 99,9% en poids d'acide thioglycolique, ou l'un de ses sels par rapport au poids total de ladite composition B ;
b) du thioglycolate d'isopropyle (IPTG) en une quantité comprise entre strictement supérieur à 0 et 130 ppm, de préférence entre 5 et 80 ppm, et plus préférentiellement entre 30 et 70 ppm ; et
c) de l'éther diisopropylique (IPE) en une quantité comprise entre 5 et 100 ppm, de préférence entre 10 et 80 ppm.

Selon un mode de réalisation, ladite composition B est obtenue selon un procédé comprenant les étapes suivantes :
a) mélange de l'acide monochloroacétique (AMCA) ou l'un de ses sels avec NaSH ou NH₄SH en milieu aqueux ;
b) acidification du mélange réactionnel obtenu à l'étape a) pour obtenir de l'acide thioglycolique, de préférence ladite acidification étant réalisée par ajout d'acide chlorhydrique ;
c) extraction de l'acide thioglycolique obtenu à l'étape b) de la phase aqueuse avec de l'éther diisopropylique ;
d) évaporation de l'éther diisopropylique de la phase organique obtenue à l'étape c) ; et
e) purification, de préférence par distillation sous vide, de l'acide thioglycolique.

### Solvant S et autres additifs

Selon un mode de réalisation, ladite composition C comprend de l'eau ou un alcool en tant que solvant. La composition C est généralement une formulation aqueuse qui peut être préparée sous forme de mélange concentré qui est dilué par l'utilisateur final. En variante, la composition C peut également être une formulation prête à l'emploi, c'est-à-dire qui ne nécessite pas d'être diluée.

La composition C peut éventuellement comprendre un ou plusieurs additifs par exemple agents rhéologiques ou de texture, agents épaississants, tensio-actifs, moussants, antimoussants, et autres connus de l'homme du métier.

### Utilisations selon l'invention

La présente invention a également pour objet l'utilisation d'un acide organique A tel que défini ci-dessus, pour masquer, diminuer ou supprimer l'odeur d'une composition B telle que définie ci-dessus. On peut considérer l'acide organique A comme un agent masquant d'odeur pour la composition B.

Selon un mode de réalisation, ladite utilisation permet de masquer, diminuer ou supprimer l'odeur soufrée et/ou de grillé et/ou de brûlé de ladite composition B telle que définie ci-dessus.

Selon un mode de réalisation, ladite utilisation selon l'invention permet de diminuer l'intensité de l'odeur et/ou d'augmenter le caractère hédonique de l'odeur de ladite composition B telle que définie ci-dessus. Selon un mode de réalisation, ladite utilisation selon l'invention permet d'obtenir une intensité de l'odeur plus faible et/ou un caractère hédonique plus important de la composition C, par rapport à la composition B telles que définies ci-dessus.

La présente invention concerne également l'utilisation d'une composition C selon l'invention dans le domaine cosmétique (par exemple dans les shampoings, les compositions pour frisage ou défrisage des cheveux et les crèmes dépilatoires), le traitement du cuir, les solutions de nettoyage, ou encore dans la préparation de polymères en tant qu'agent de transfert de chaîne.

La présente invention concerne également un kit comprenant une composition B telle que définie ci-dessus et un acide organique A tel que défini ci-dessus.

### Procédé de préparation de la composition C selon l'invention

La présente invention a pour objet un procédé de préparation d'une composition C telle que définie ci-dessus, caractérisé en ce qu'il comprend une étape de mélange d'au moins un acide organique A tel que défini ci-dessus et d'une composition B telle que définie ci-dessus. La préparation d'une telle composition C peut être réalisée de façon connue par l'homme du métier, par simple mélange de la composition B avec au moins un acide organique A tels que définis ci-dessus.

Selon un mode de réalisation, ladite composition **C** comprend :
- au moins un acide organique A, linéaire ou cyclique, comprenant de 1 à 3 fonction(s) acide carboxylique et comprenant une chaîne hydrocarbonée saturée ou insaturée ; linéaire ou ramifiée ou cyclique contenant de 1 à 10 atome(s) de carbone ;
   lesdits atome(s) de carbone étant éventuellement substitué(s) par un ou plusieurs substituant(s) choisi(s) parmi -OH et =O ; ou l'un de ses sels ;
- de l'acide thioglycolique (ATG), ou l'un de ses sels ou de ses esters ; et
- du thioglycolate d'isopropyle (IPTG) en une quantité comprise entre strictement supérieur à 0 et 130 ppm, de préférence entre 5 et 80 ppm, et plus préférentiellement entre 30 et 70 ppm par rapport à la quantité d'ATG ; et
- éventuellement un solvant S ;
lesdits acide(s) organique(s) A et acide thioglycolique étant en des proportions telles que le ratio molaire (acide(s) organique(s) A/acide thioglycolique) est compris entre 0,0004 et 0,1 ; de préférence entre 0,0004 et 0,004.

### EXEMPLES

### Exemple 1 : Tests olfactifs menés sur des compositions d'acide thioglycolique

### I. Préparation des échantillons :

On prépare une composition B selon le procédé suivant :
De l'acide monochloroacétique (1 équivalent) est mis à réagir avec du sulfhydrate d'ammonium (2 équivalents) dans un réacteur tubulaire à une température de 15 à 100°C sous une pression de 5 à 25 bars d'hydrogène sulfuré. Le produit obtenu est ensuite acidifié à l'acide chlorhydrique 33% jusqu'à obtention d'un pH acide puis l'acide thioglycolique est extrait à l'éther diisopropylique (IPE). L'IPE est ensuite évaporé puis l'ATG est distillé.

La composition B obtenue présente les caractéristiques suivantes :

| **Composé** | **Unité** | **Quantité** |
|---|---|---|
| ATG | % | 99,3 |
| IPE | ppm | 17 |
| AMCA | ppm | <0,05 |
| IPTG | ppm | 60 |

Puis, 100g de cette composition B ont été mélangés avec les acides organiques A suivants et 15 mL de chacune des solutions obtenues a subi une analyse olfactive. Ces compositions correspondent aux compositions C selon l'invention.

| | **Acide organique A ajouté** | | **Ratio molaire A/ATG** |
|---|---|---|---|
| N°échantillon | Quantité (ppm) | Type | |
| 1 | - | - | |
| 2 | 500 | acétique | 0,0008 |
| 5 | 2000 | acétique | 0,0031 |
| 6 | 1000 | glycolique | 0,0012 |
| 7 | 1000 | lactique | 0,0010 |
| 8 | 1000 | propionique | 0,0012 |
| 9 | 1000 | citrique | 0,0005 |
| 10 | 1000 | salicylique | 0,0007 |
| 11 | 1000 | cyclohexanecarboxylique | 0,0007 |
| 12 | 1000 | 2-éthylbutyrique | 0,0008 |
| 13 | 1000 | pyruvique | 0,0010 |
| 14 | 1000 | lévulinique | 0,0008 |
| 16 | 1000 | ascorbique | 0,0005 |
| 17 | 1000 | acétonedicarboxylique | 0,0006 |
| 18 | 1000 | 2-céto-L-gluconique | 0,0005 |

### II. Méthode d'échantillonnage et méthode analytique :

### Echantillonnage :

Pour chaque échantillon, un volume de 0,1 µL a été prélevé de manière à obtenir une concentration en phase gazeuse de 1 ppm soit 4,5 mg.m⁻³. Ce volume a été introduit dans une poche Nalophan^{™} puis un gaz neutre (air) a été ajouté à un débit contrôlé (cf. EN 13725, §6.2 et 6.4). Les poches ont ensuite été mises à l'étuve pendant 1h à 25°C.

Les concentrations introduites et les paramètres d'échantillonnage sont les suivants :

| Echantillons | Température | Temps d'éch. | Vol inj en µL | Densité | Concentration en mg.m⁻³ |
|---|---|---|---|---|---|
| Tous les échantillons | 25°C | 20 min | 0,1 | 1,33 | 4,5 |

### III. Analyses sensorielles :

### 1. Réalisation des analyses sensorielles :

Les analyses sensorielles ont été réalisées le jour même de l'échantillonnage avec un panel de jurés de nez et olfactomètre tels que décrits par la norme EN 13725 (§6). Le débit est d'au moins 20 l/min. Le temps accordé pour évaluer le stimulus présenté ne dépasse pas les 15 s. L'intervalle de temps entre les stimuli doit être suffisant pour éviter l'adaptation des jurés de nez à l'odeur (§8 EN 13725).

Après perception, les panélistes ont ensuite indiqué leur ressenti selon les paramètres et échelles indiqués ci-dessous :
a) *Intensité :*
   Les mesures d'intensité, basées sur une norme allemande (VDI 3882 part 1), ont été réalisées avec un panel de 6 personnes sélectionnées et entrainées à partir d'une échelle de référence de n-butanol selon la norme EN 13725 (§6.4.2).

Lors des mesures, les jurés de nez sentent directement le produit échantillonné et évaluent son intensité sur une échelle de 0 à 5.

| Intensité de l'odeur | Echelle |
|---|---|
| Très fort | 5 |
| Fort | 4 |
| Moyen | 3 |
| Faible | 2 |
| Très faible | 1 |
| Pas détectable | 0 |

L'intensité moyenne de la composition est calculée selon la moyenne arithmétique de chaque résultat individuel.

### b) Caractère hédonique :

Les mesures de caractère hédonique, basées sur une norme allemande (VDI 3882 part 2), ont également été réalisées avec un panel de 6 personnes sélectionnées selon la norme EN 13725.

Sur chaque échantillon, les jurés donnent leur ressenti sur le caractère agréable ou désagréable de l'odeur selon une échelle de -4 à +4.

| Caractère hédonique | Echelle |
|---|---|
| Extrêmement agréable | +4 |
| Très agréable | +3 |
| Agréable | +2 |
| Faiblement agréable | +1 |
| Neutre | 0 |
| Faiblement désagréable | -1 |
| Désagréable | -2 |
| Très désagréable | -3 |
| Extrêmement désagréable | -4 |

### c) Caractérisation de l'odeur :

La caractérisation des odeurs est réalisée par des jurés de nez experts.

### IV. Résultats :

Le tableau ci-dessus présente les résultats moyennés des 6 panélistes pour les 18 échantillons testés, en intensité et en caractère hédonique, à 25°C.

| N°échantillon | Type d'acide organique A | Moyenne Intensité | Moyenne caractère hédonique |
|---|---|---|---|
| **1** | - (ATG seul) | 3,33 | -0,33 |
| 2 | acétique | 2,83 | 0,17 |
| 5 | acétique | 3,33 | -0,17 |
| 6 | glycolique | 2,83 | -0,33 |
| 7 | lactique | 3,00 | -0,33 |
| 8 | propionique | 3,00 | 0,17 |
| 9 | citrique | 2,67 | -0,17 |
| 10 | Salicylique | 3,00 | -0,50 |
| 11 | Cyclohexanecarboxylique | 3,17 | -0,67 |
| 12 | 2-éthylbutyrique | 2,83 | 1,00 |
| 13 | pyruvique | 2,83 | 0,00 |
| 14 | Lévulinique | 2,83 | -0,50 |
| 16 | ascorbique | 3,17 | -0,17 |
| 17 | acétonedicarboxylique | 3,17 | 0,67 |
| 18 | 2-céto-Lgluconique | 4,17 | 0,33 |

### 1. Description de l'intensité et du caractère hédonique obtenu :

L'échantillon 1 d'ATG non traité par un acide organique A présente une intensité moyenne de 3,33 et un caractère hédonique de -0,33.

On observe que l'ajout d'un acide organique A à une composition d'ATG comprenant 60 ppm d'IPTG permet d'obtenir une intensité plus faible et/ou un caractère hédonique plus élevé par rapport à une même composition d'ATG sans ajout d'acide organique A.

On observe des intensités comprises entre 2,83 et 4,17 et des appréciations hédoniques comprises entre -0,67 et 1.

Les valeurs se situant autour de 0, notamment entre -0,17 et 1, décrivent des odeurs neutres, voire agréables.

L'échantillon perçu comme le plus intense (4,17 soit entre fort et très fort) est l'échantillon 18. Le moins intense est l'échantillon 9 avec 2,67 (faible-moyen).

Concernant le caractère hédonique, 9 échantillons sur les 18 sont considérés comme relativement neutres avec des valeurs autour de 0 (de -0,17 à +0,17) et 3 échantillons comme légèrement agréables (échantillons 12, 17 et 18).

### 2. Profils sensoriels de tous les échantillons :

Globalement, les échantillons considérés comme les plus désagréables (10, 11 et 14) ont tous été décrits avec une note soufrée importante.

Les échantillons considérés comme les plus agréables sont caractérisé par une note soufrée faible (échantillon 12) ou absente (échantillons 17 et 18) et une note rance / acide relativement faible. L'ajout d'un acide organique A selon l'invention permet donc de masquer, diminuer voire supprimer la note soufrée de l'ATG produit industriellement. Les notes de grillé et/ou brûlé sont également plus faibles lorsque l'ATG produit industriellement est additivé selon l'invention.

## Revendications

1. Composition **C** comprenant :
- au moins un acide organique **A,** linéaire ou cyclique, comprenant de 1 à 3 fonction(s) acide carboxylique et comprenant une chaîne hydrocarbonée saturée ou insaturée; linéaire ou ramifiée ou cyclique contenant de 1 à 10 atome(s) de carbone ;
lesdits atome(s) de carbone étant éventuellement substitué(s) par un ou plusieurs substituant(s) choisi(s) parmi -OH et =O ; ou l'un de ses sels ;
- une composition **B** comprenant :
a/ de l'acide thioglycolique ou l'un de ses sels ou de ses esters ; et
b/ du thioglycolate d'isopropyle (IPTG) en une quantité comprise entre strictement supérieur à 0 et 130 ppm, de préférence entre 5 et 80 ppm, et plus préférentiellement entre 30 et 70 ppm ; et
- éventuellement un solvant **S** ;
lesdits acide(s) organique(s) A et acide thioglycolique étant en des proportions telles que le ratio molaire (acide(s) organique(s) A/acide thioglycolique) est compris entre 0,0004 et 0,1 ; de préférence entre 0,0004 et 0,004.

2. Composition selon la revendication 1, dans laquelle ledit au moins un acide organique A est l'acide ascorbique ou un acide organique de formule générale (I) suivante : 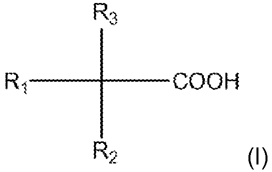 dans laquelle :
- R₁ est choisi parmi le groupe constitué de : -H, -OH, (Ci-Cio)alkyle et (C₂-C₁₀)alcényle ;
lesdits (Ci-Cio)alkyle et (C₂-C₁₀)alcényle étant éventuellement substitué(s) par au moins un substituant choisi parmi le groupe constitué de : -OH, -C(O)OH et =O ;
- R₂ est -H, -OH ou (Ci-Cio)alkyle ;
- R₃ est H ;
ou R₂ et R₃ forment ensemble avec l'atome de carbone auxquels ils se rattachent un groupement C=O ;
ou R₁ et R₂ forment ensemble avec l'atome de carbone auxquels ils se rattachent un groupement (C₃-C₁₀)cycloalkyle ou (C₆-C₁₀)aryle,
lesdits groupements (C₃-C₁₀)cycloalkyle et (C₆-C₁₀)aryle étant éventuellement substitué(s) par au moins un substituant choisi parmi le groupe constitué de :
-OH, -C(O)OH et =O ;
ou l'un de ses sels.

3. Composition selon la revendication 1 ou 2, dans laquelle l'acide organique A est choisi dans le groupe constitué de l'acide acétique, l'acide glycolique, l'acide lactique, l'acide propionique, l'acide citrique, l'acide salicylique, l'acide cyclohexanecarboxylique, l'acide 2-éthylbutyrique, l'acide pyruvique, l'acide lévulinique, l'acide ascorbique, l'acide acétonedicarboxylique, l'acide 2-céto-L-gluconique, et leurs mélanges.

4. Composition selon l'une quelconque des revendications précédentes, dans laquelle l'acide organique A est choisi parmi l'acide acétique et l'acide 2-éthylbutyrique, de préférence l'acide acétique.

5. Composition selon l'une quelconque des revendications précédentes, dans laquelle la composition B comprend :
a/ de l'acide thioglycolique ou l'un de ses sels ;
b/ du thioglycolate d'isopropyle (IPTG) en une quantité comprise entre strictement supérieur à 0 et 130 ppm, de préférence entre 5 et 80 ppm, et plus préférentiellement entre 30 et 70 ppm ; et
c/ de l'éther diisopropylique (IPE) en une quantité comprise entre 5 et 100 ppm, de préférence entre 10 et 80 ppm.

6. Composition selon l'une quelconque des revendications précédentes, dans laquelle le ratio molaire (acide(s) organique(s) A/acide thioglycolique) est compris entre 0,0008 et 0,008.

7. Composition selon l'une quelconque des revendications précédentes, comprenant entre 50% et 99,9% en poids d'acide thioglycolique par rapport au poids total de ladite composition.

8. Composition selon l'une quelconque des revendications 1 à 7, **caractérisée en ce que** ladite composition B est obtenue selon un procédé comprenant les étapes suivantes :
a/ mélange de l'acide monochloroacétique (AMCA) ou l'un de ses sels avec NaSH ou NH₄SH en milieu aqueux ;
b/ acidification du mélange réactionnel obtenu à l'étape a) pour obtenir de l'acide thioglycolique, de préférence ladite acidification étant réalisée par ajout d'acide chlorhydrique ;
c/ extraction de l'acide thioglycolique obtenu à l'étape b) de la phase aqueuse avec de l'éther diisopropylique ;
d/ évaporation de l'éther diisopropylique de la phase organique obtenue à l'étape c) ; et
e/ purification, de préférence par distillation sous vide, de l'acide thioglycolique.

9. Utilisation d'au moins un acide organique A tel que défini à l'une quelconque des revendications 1 à 4, pour masquer, diminuer ou supprimer l'odeur d'une composition B comprenant :
a/ de l'acide thioglycolique ou l'un de ses sels ; et
b/ du thioglycolate d'isopropyle (IPTG) en une quantité comprise entre strictement supérieur à 0 et 130 ppm, de préférence entre 5 et 80 ppm, et plus préférentiellement entre 30 et 70 ppm.

10. Utilisation d'une composition C selon l'une quelconque des revendications 1 à 8, dans le domaine cosmétique, le traitement du cuir, les solutions de nettoyage, ou dans la préparation de polymères en tant qu'agent de transfert de chaîne.

11. Kit comprenant une composition B telle que définie à l'une quelconque des revendications 1, 5 ou 8 et un acide organique A tel que défini à l'une quelconque des revendications 1 à 4.

12. Procédé de préparation d'une composition C selon l'une quelconque des revendications 1 à 8, **caractérisé en ce qu'**il comprend une étape de mélange d'au moins un acide organique A tel que défini à l'une quelconque des revendications 1 à 4 et d'une composition B comprenant :
a/ de l'acide thioglycolique ou l'un de ses sels ; et
b/ du thioglycolate d'isopropyle (IPTG) en une quantité comprise entre strictement supérieur à 0 et 130 ppm, de préférence entre 5 et 80 ppm, et plus préférentiellement entre 30 et 70 ppm.

## Patentansprüche

1. Zusammensetzung **C,** umfassend:
- mindestens eine lineare oder cyclische organische Säure **A** mit 1 bis 3 Carbonsäurefunktionen und einer gesättigten oder ungesättigten linearen oder verzweigten oder cyclischen Kohlenwasserstoffkette mit 1 bis 10 Kohlenstoffatomen;
wobei die Kohlenstoffatome gegebenenfalls durch einen oder mehrere Substituenten, die aus -OH und =O ausgewählt sind, substituiert sind; oder ein Salz davon;
- eine Zusammensetzung **B,** umfassend:
a/ Thioglykolsäure oder ein Salz davon oder ein Ester davon und
b/ Isopropylthioglykolat (IPTG) in einer Menge zwischen streng größer als 0 und 130 ppm, vorzugsweise zwischen 5 und 80 ppm und weiter bevorzugt zwischen 30 und 70 ppm; und
- gegebenenfalls Lösungsmittel **S;**
wobei die organische Säure bzw. die organischen Säuren A und Thioglykolsäure in solchen Anteilen vorliegen, dass das Molverhältnis von organischer Säure bzw. organischen Säuren A zu Thioglykolsäure zwischen 0,0004 und 0,1, vorzugsweise zwischen 0,0004 und 0,004, liegt.

2. Zusammensetzung nach Anspruch 1, wobei es sich bei der mindestens einen organischen Säure A um Ascorbinsäure oder eine organische Säure der folgenden allgemeinen Formel (I): 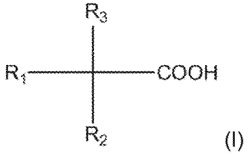 wobei:
- R₁ aus der Gruppe bestehend aus -H, -OH, (C₁-C₁₀)Alkyl und (C₂-C₁₀) Alkenyl ausgewählt ist;
wobei das (C₁-C₁₀)Alkyl und (C₂-C₁₀)Alkenyl gegebenenfalls durch mindestens einen Substituenten aus der Gruppe bestehend aus -OH, -C(O)OH und =O substituiert sind;
- R₂ für -H, -OH oder (C₁-C₁₀)Alkyl steht;
- R₃ für H steht;
oder R₂ und R₃ zusammen mit dem Kohlenstoffatom, an das sie gebunden sind, eine C=O-Gruppe bilden;
oder R₁ und R₂ zusammen mit dem Kohlenstoffatom, an das sie gebunden sind, eine (C₃-C₁₀)Cycloalkyl- oder (C₆-C₁₀)Arylgruppe bilden,
wobei die (C₃-C₁₀) Cycloalkyl- und (C₆-C₁₀) Arylgruppen gegebenenfalls durch mindestens einen Substituenten aus der Gruppe bestehend aus -OH, -C(O)OH und =O substituiert sind;
oder ein Salz davon handelt.

3. Zusammensetzung nach Anspruch 1 oder 2, wobei die organische Säure A aus der Gruppe bestehend aus Essigsäure, Glykolsäure, Milchsäure, Propionsäure, Citronensäure, Salicylsäure, Cyclohexancarbonsäure, 2-Ethylbuttersäure, Brenztraubensäure, Lävulinsäure, Ascorbinsäure, Acetondicarbonsäure, 2-Keto-L-gluconsäure und Mischungen davon ausgewählt ist.

4. Zusammensetzung nach einem der vorhergehenden Ansprüche, wobei die organische Säure A aus Essigsäure und 2-Ethylbuttersäure, vorzugsweise Essigsäure, ausgewählt ist.

5. Zusammensetzung nach einem der vorhergehenden Ansprüche, wobei die Zusammensetzung B Folgendes umfasst:
a/ Thioglykolsäure oder ein Salz davon;
b/ Isopropylthioglykolat (IPTG) in einer Menge zwischen streng größer als 0 und 130 ppm, vorzugsweise zwischen 5 und 80 ppm und weiter bevorzugt zwischen 30 und 70 ppm; und
c/Diisopropylether (IPE) in einer Menge zwischen 5 und 100 ppm, vorzugsweise zwischen 10 und 80 ppm.

6. Zusammensetzung nach einem der vorhergehenden Ansprüche, wobei das Molverhältnis von organischer Säure bzw. organischen Säuren A zu Thioglykolsäure zwischen 0,0008 und 0,008 liegt.

7. Zusammensetzung nach einem der vorhergehenden Ansprüche, umfassend zwischen 50 und 99,9 Gew.-% Thioglykolsäure, bezogen auf das Gesamtgewicht der Zusammensetzung.

8. Zusammensetzung nach einem der Ansprüche 1 bis 7, **dadurch gekennzeichnet, dass** die Zusammensetzung B gemäß einem Verfahren erhalten wird, das die folgenden Schritte umfasst:
a/ Mischen von Monochloressigsäure (MCES) oder einem Salz davon mit NaSH oder NH₄SH in wässrigem Medium;
b/ Ansäuern der in Schritt a) erhaltenen Reaktionsmischung zum Erhalt von Thioglykolsäure, wobei das Ansäuern vorzugsweise durch Zugabe von Chlorwasserstoffsäure durchgeführt wird;
c/ Extrahieren der in Schritt b) erhaltenen Thioglykolsäure aus der wässrigen Phase mit Diisopropylether;
d/ Verdampfen des Diisopropylethers aus der in Schritt c) erhaltenen organischen Phase und
e/ Reinigen der Thioglykolsäure, vorzugsweise durch Vakuumdestillation.

9. Verwendung von mindestens einer organischen Säure A gemäß einem der Ansprüche 1 bis 4 zur Maskierung, Abschwächung oder Unterdrückung des Geruchs einer Zusammensetzung B, umfassend:
a/ Thioglykolsäure oder ein Salz davon und
b/ Isopropylthioglykolat (IPTG) in einer Menge zwischen streng größer als 0 und 130 ppm, vorzugsweise zwischen 5 und 80 ppm und weiter bevorzugt zwischen 30 und 70 ppm.

10. Verwendung einer Zusammensetzung C nach einem der Ansprüche 1 bis 8 auf dem Gebiet der Kosmetik, bei der Lederbehandlung, in Reinigungslösungen oder bei der Herstellung von Polymeren als Kettenübertragungsmittel.

11. Kit, umfassend eine Zusammensetzung B gemäß einem der Ansprüche 1, 5 oder 8 und eine organische Säure A gemäß einem der Ansprüche 1 bis 4.

12. Verfahren zur Herstellung einer Zusammensetzung C gemäß einem der Ansprüche 1 bis 8, **dadurch gekennzeichnet, dass** es einen Schritt des Mischens mindestens einer organischen Säure A gemäß einem der Ansprüche 1 bis 4 und einer Zusammensetzung B, umfassend:
a/ Thioglykolsäure oder ein Salz davon und
b/ Isopropylthioglykolat (IPTG) in einer Menge zwischen streng größer als 0 und 130 ppm, vorzugsweise zwischen 5 und 80 ppm und weiter bevorzugt zwischen 30 und 70 ppm,
umfasst.

## Claims

1. Composition **C** comprising:
- at least one linear or cyclic organic acid **A** comprising from 1 to 3 carboxylic acid functions and comprising a saturated or unsaturated, linear or branched or cyclic hydrocarbon chain containing from 1 to 10 carbon atoms; said carbon atoms being optionally substituted by one or more substituents chosen from -OH and =O; or one of its salts;
- a composition **B** comprising:
a/ thioglycolic acid or one of its salts or esters; and
b/ isopropyl thioglycolate (IPTG) in an amount of between strictly greater than 0 and 130 ppm, preferably between 5 and 80 ppm, and more preferentially between 30 and 70 ppm; and
- optionally a solvent ***S*;**
said organic acid(s) A and thioglycolic acid being in proportions such that the (organic acid(s) A/thioglycolic acid) molar ratio is between 0.0004 and 0.1, preferably between 0.0004 and 0.004.

2. Composition according to Claim 1, wherein said at least one organic acid A is ascorbic acid or an organic acid of the following general formula (I): 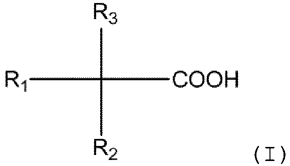 in which:
- R₁ is chosen from the group consisting of: -H, -OH, (C₁-C₁₀) -alkyl and (C₂-C₁₀) -alkenyl;
said (C₁-C₁₀)-alkyl and (C₂-C₁₀) -alkenyl being optionally substituted by at least one substituent chosen from the group consisting of: -OH, -C(O)OH and =O;
- R₂ is -H, -OH or (C₁-C₁₀) -alkyl;
- R₃ is H;
or R₂ and R₃ form, together with the carbon atom to which they are attached, a C=O group;
or R₁ and R₂ form, together with the carbon atom to which they are attached, a (C₃-C₁₀) -cycloalkyl or (C₆-C₁₀) -aryl group,
said (C₃-C₁₀) -cycloalkyl and (C₆-C₁₀)-aryl groups being optionally substituted by at least one substituent chosen from the group consisting of:
-OH, -C(O)OH and =O;
or one of its salts.

3. Composition according to Claim 1 or 2, wherein the organic acid A is chosen from the group consisting of acetic acid, glycolic acid, lactic acid, propionic acid, citric acid, salicylic acid, cyclohexanecarboxylic acid, 2-ethylbutyric acid, pyruvic acid, levulinic acid, ascorbic acid, acetonedicarboxylic acid, 2-keto-L-gluconic acid, and mixtures thereof.

4. Composition according to any one of the preceding claims, wherein the organic acid A is chosen from acetic acid and 2-ethylbutyric acid, preferably acetic acid.

5. Composition according to any one of the preceding claims, wherein the composition B comprises:
a/ thioglycolic acid or one of its salts;
b/ isopropyl thioglycolate (IPTG) in an amount of between strictly greater than 0 and 130 ppm, preferably between 5 and 80 ppm, and more preferentially between 30 and 70 ppm; and
c/ diisopropyl ether (IPE) in an amount of between 5 and 100 ppm, preferably between 10 and 80 ppm.

6. Composition according to any one of the preceding claims, wherein the (organic acid(s) A/thioglycolic acid) molar ratio is between 0.0008 and 0.008.

7. Composition according to any one of the preceding claims, comprising between 50% and 99.9% by weight of thioglycolic acid relative to the total weight of said composition.

8. Composition according to any one of Claims 1 to 7, **characterized in that** said composition B is obtained by a process comprising the following steps:
a/ mixing monochloroacetic acid (MCAA) or one of its salts with NaSH or NH₄SH in an aqueous medium;
b/ acidifying the reaction mixture obtained in step a) to obtain thioglycolic acid, said acidification preferably being performed by addition of hydrochloric acid;
c/ extracting the thioglycolic acid obtained in step b) from the aqueous phase with diisopropyl ether;
d/ evaporating the diisopropyl ether from the organic phase obtained in step c); and
e/ purifying, preferably by vacuum distillation, the thioglycolic acid.

9. Use of at least one organic acid A as defined in any one of Claims 1 to 4 for masking, reducing or eliminating the odor of a composition B comprising:
a/ thioglycolic acid or one of its salts; and
b/ isopropyl thioglycolate (IPTG) in an amount of between strictly greater than 0 and 130 ppm, preferably between 5 and 80 ppm, and more preferentially between 30 and 70 ppm.

10. Use of a composition C according to any one of Claims 1 to 8 in the cosmetics field, leather treatment, cleaning solutions or in the preparation of polymers as chain-transfer agent.

11. Kit comprising a composition B as defined in any one of Claims 1, 5 or 8 and an organic acid A as defined in any one of Claims 1 to 4.

12. Process for preparing a composition C according to any one of Claims 1 to 8, **characterized in that** it comprises a step of mixing at least one organic acid A as defined in any one of Claims 1 to 4 and a composition B comprising:
a/ thioglycolic acid or one of its salts; and
b/ isopropyl thioglycolate (IPTG) in an amount of between strictly greater than 0 and 130 ppm, preferably between 5 and 80 ppm, and more preferentially between 30 and 70 ppm.
